# EUROPEAN PATENT SPECIFICATION

(11) **EP 1 347 299 B1**
(45) Date of publication and mention of the grant of the patent: **08.03.2006**
(21) Application number: 01272323.5
(22) Date of filing: 25.12.2001
(51) Int. Cl.: G01N 33/543

(54) **SPECIFIC BONDING ANALYSIS METHOD AND SPECIFIC BONDING ANALYSIS DEVICE USING IT**
ANALYSEVERFAHREN DURCH SPEZIFISCHES BINDEN UND VORRICHTUNG, DIE DAS VERFAHREN EINSETZT
PROCEDE D'ANALYSE DE LIAISON SPECIFIQUE ET DISPOSITIF D'ANALYSE DE LIAISON SPECIFIQUE CORRESPONDANT

(30) Priority: 26.12.2000 JP 2000394294; 27.06.2001 JP 2001194081
(43) Date of publication of application: 24.09.2003
(73) Proprietor: MATSUSHITA ELECTRIC INDUSTRIAL CO., LTD., Kadoma-shi, Osaka 571-8501 (JP)
(72) Inventor: KENJYOU, Noriko, Hirakata-shi, Osaka 573-0036 (JP); KAMEI, Akihito, Yawata-shi, Kyoto 614-8295 (JP); KAWAMURA, Tatsurou, Kyotanabe-shi, Kyoto 610-0351 (JP); NADAOKA, Masataka, Iyo-shi, Ehime 799-3113 (JP); TAKAHASHI, Mie, Niihama-shi, Ehime 792-0026 (JP); TANAKA, Hirotaka, Matsuyama-shi, Ehime 791-1102 (JP)
(74) Representative: TBK-Patent
(86) International application number: PCT/JP2001/011415
(87) International publication number: WO 2002/052265

(56) References cited:
- WO-A-97/09620
- WO-A-99/22236
- JP-A- 3 110 469
- JP-A- 6 094 717
- JP-A- 8 240 591
- JP-A- 11 038 006
- JP-A- 61 280 568
- JP-A- 2000 221 195

## Description

### Technical Field

The present invention relates to a specific binding analysis method of quantitatively or qualitatively determining an analyte in a sample, and to a specific binding analysis apparatus used therefor.

### Background Art

With the recent expansion of medical care in households and communities as well as increase of clinical examinations requiring high urgency, there is an increasing demand for the development of a specific binding analysis method which can be performed even by persons other than the experts of the clinical examination, in a rapid, simple and accurate manner.

Many methods are known as the conventional specific binding analyses, which include immunoassay utilizing an antigen-antibody reaction, receptor assay employing a receptor and nucleic acid probe assay employing the hybridization of complementary nucleic acid sequences. Because of their high specificity, these methods are being widely used in the clinical examinations and in many other fields.

In chromatography, which is a type of immunoassay, a liquid sample is contacted with a matrix comprising, for example, a porous carrier or a fine particle-packed carrier in each of which a specific binding substance is insolubilized (immobilized), and the presence or absence of an analyte in the sample is analyzed by utilizing a phenomenon in which the liquid sample permeates along the matrix by permeating force caused by capillarity (see Japanese Patent Nos. 2504923 and 2667793, Japanese Examined Patent Publication No. Hei 7-78503, Japanese Unexamined Patent Publication Nos. Hei 10-73592 and Hei 8-240591).

More specifically, a first specific binding substance, which is labeled with a labeling material freely detectable by naked eyes or with an optical method, is specifically bound to an analyte first. Subsequently, the analyte is bound, via the first specific binding substance, to a binding material immobilized on the matrix. Then, finally, the presence or absence of the analyte in the sample is analyzed, according to the labeled amount of the first specific binding substance immobilized on the matrix.

The carrier comprising the matrix used for such chromatography has a large surface area where a great amount of a specific binding substance can be immobilized, so that the collision between reacting molecules that may cause a specific binding reaction, occurs with a higher frequency as compared with the reaction in a liquid phase. Accordingly, the above-described chromatography is advantageous from the viewpoint of the measurement sensitivity and the measurement time.

However, the above-described chromatography has the first problem called a prozone phenomenon. This is a problem occurring mainly when the concentration of an analyte in a sample is high. The prozone phenomenon is a phenomenon in which the concentration of an analyte cannot be unambiguously determined from a signal intensity attributed to a specific binding reaction.

Specifically, when an excessive amount of an analyte is present in a sample, there are present on a matrix an analyte bound to a labeled first specific binding substance, and an analyte (simple substance) not bound to the labeled first specific binding substance. The analyte bound to the labeled first specific binding substance and the analyte as the simple substance compete to specifically bind to a binding material (second specific binding substance) immobilized on the matrix. Thus, there is a case where the analyte as the simple substance undesirably binds to the binding material, and the analyte bound to the labeled first specific binding substance migrates beyond a detection zone to be flowed out from the detection zone due to permeating force caused by capillarity. This results in a reduced amount of the labeled first specific binding substance bound to the binding material, so that the signal intensity attributed to the specific binding reaction of the first specific binding substance does not precisely correspond to the amount of the analyte contained in the sample.

Accordingly, when an excessive amount of an analyte is present in a sample, the amount of the analyte determined by the labeled amount is abnormally reduced, thereby often preventing an accurate determination of the presence or absence and concentration of the analyte in the sample.

In order to accurately measure the concentration and the like of an analyte by eliminating the influence of the prozone phenomenon, there has been proposed a method of confirming the presence or absence of an analyte at a high concentration by diluting a sample to various concentrations and individually measuring a plurality of signals of the sample at various concentrations. However, this requires the use of a plurality of reaction vessels and therefore renders the measurement steps complicated, resulting in a problem of increasing the size and complexity of the analysis apparatus.

Therefore, it is a first object of the present invention to provide a specific binding analysis method capable of quantitatively or qualitatively determining an analyte in a sample in a rapid, simple and accurate manner with little influence of the prozone phenomenon, and a specific binding analysis apparatus used therefor.

The above-described conventional chromatography has the second problem of the influence of a background. This is a problem occurring mainly when the concentration of an analyte in a sample is low. The background refers to a behavior exhibited by a sample containing no analyte. For example, a signal intensity attributed to the nonspecific absorption of a specific binding substance or the like, and to coloration or the like of the sample itself in the case of measuring a signal attributed to the coloration, is added to the signal intensity of the analyte itself, thereby reducing the sensitivity of the measurement. In other words, the signal intensity of the background is generally considered as zero, but it becomes greater than zero owing to the nonspecific absorption of a specific binding substance or the like and to the coloration of the sample itself in the case of measuring a signal attributed to the coloration.

In the case where a nonspecific absorption occurs, in order to reduce the nonspecific absorption, for example, a reaction site is pre-treated by coating it with a surfactant, a blocking material or the like, and then a sample is added thereto, followed by washing the reaction site. However, a certain degree of nonspecific absorption is present even after the above-described operations, and therefore, it is difficult to completely eliminate the influence of the background.

Or, in the case of, for example, immunochromatography involving developing a substance that exhibits coloration by itself, such as whole blood, which is frequently required as a sample for clinical examinations, a signal attributed to the coloration in the detection zone includes a signal attributed to the background, in addition to a signal attributed to a specific binding reaction. Moreover, it is not easy to detect only a signal attributed to the specific binding reaction until the flow of the sample causes a colored component in the sample to pass the detection zone, i.e., to exit from the detection zone. Therefore, the detection of the signal needs to be delayed until the signal attributed to the background becomes negligible, resulting in a problem of requiring a long time for the measurement.

In view of this, there have been proposed various methods for reducing the influence of the background, including a pre-treatment of the sample by centrifugation and the like, removal of a colored component contained in the sample by filtration and the like, or study of wavelength to be used. However, these methods introduce additional problems such as an increase in the complexity of the operations, a reduction in the development of a sample in a chromatograph and a decrease in the signal intensity in the detection zone.

There is another available method which involves reading a difference between a signal intensity attributed only to the background and a signal intensity in the detection zone, by scanning an immunochromatograph or an external measurement instrument; however, this leads to an increase in the size and complexity of the measurement apparatus.

Furthermore, there is a problem common to the specific binding analysis methods, that is, the influence of a foreign matter in a sample. The foreign matter refers to a substance other than an analyte contained in a sample, such as an impurity. The foreign matter binds to an analyte or to a substance exhibiting a similar behavior to that of the analyte (e.g., an analog of the analyte), thereby impeding a specific binding reaction between the analyte and the specific binding substance. Thus, a signal intensity actually detected from the labeling in the detection zone is attributed to a specific binding reaction between each of the analyte and the foreign matter with the specific binding substance. Accordingly, the presence of the foreign matter has a great influence on a result of the quantitative or qualitative determination in a specific binding analysis method.

When the foreign matter is removed from the sample by a pre-treatment in order to reduce the influence of the foreign matter, complex operations are required. Although there is another method in which a substance specifically binding only to an analyte is selected as the specific binding substance, the selection of such a specific binding substance itself is often difficult depending on the analyte.

Therefore, it is a second object of the present invention to provide a specific binding analysis method capable of quantitatively or qualitatively determining an analyte in a sample in a simple, rapid and accurate manner with little influence of the background or foreign matter, and a specific binding analysis apparatus used therefor.

### Disclosure of Invention

The present invention provides a specific binding analysis method of quantitatively determining an analyte in a sample from a signal attributed to a specific binding reaction between the analyte in the sample and a specific binding substance capable of specifically binding to the analyte, characterized by comprising the steps of:
(a) previously preparing a database comprising, for an individual sample containing a suspected analyte,
   at least the relation between a concentration of the analyte and a saturation value or change of a signal intensity attributed to a specific binding reaction between the analyte in the sample and a specific binding substance capable of specifically binding to the analyte,
   the change over time of the signal intensity, and
   time tₛ at which the saturation value is obtained and time tₘ at which a maximum value of the signal intensity is obtained;
(b) preparing a sample containing an analyte and determining, based on the database, a measurement pattern of the signal intensity corresponding to the sample,
(c) causing a specific binding reaction between the analyte and a specific binding substance,
(d) measuring, based on the measurement pattern, the signal intensity at least twice in a period in which a signal intensity attributed to the specific binding reaction reaches saturation, after the step (c): and
(e) determining, based on the database, an amount of the analyte in the sample by using at least two signal intensities obtained in the step (d).

It is preferable that, when the sample in the step (b) has the maximum value judging from the database,
the measurement pattern comprises measurement of signal intensities A₁ and A₂ at least at times t₁ and t₂ (tₘ ≦ t₁ < t₂ ≦ tₛ), respectively, and measurement of signal intensity Aₛ at the time tₛ, and that,
in the step (e), an amount of an analyte in the sample is determined, based on the relation in the database by using the change of the signal intensities A₁ and A₂, and the signal intensity Aₛ.

It is also preferable that, when the sample in the step (b) does not have the maximum value judging from the database,
the measurement pattern comprises measurement of signal intensities A₃ and A₄ at least at times t₃ and t₄ (t₃ < t₄ < tₛ), and that,
in the step (e), an amount of an analyte in the sample is determined based on the change of the signal intensities A₃ and A4, and the relation in the database.

Further, it is preferable that, in the step (c), the analyte is specifically bound to a first specific binding substance labeled with a labeling material, and then the analyte is specifically bound to a second specific binding substance.

It is also preferable that the above-described specific binding analysis method further comprises,
prior to the step (b), step (X) of producing a strip comprising a sample application zone where the sample is applied and a detection zone where a second specific binding substance is practically immobilized and a signal attributed to a specific binding reaction can be detected,
wherein, in the step (d), the sample is applied to the sample application zone to allow the sample to flow into the detection zone by capillarity, thereby causing the analyte bound with the first specific binding substance to specifically bind to the second specific binding substance.

Also, it is preferable that, in the step (d), the signal intensity is measured by using the labeling material.

It is also preferable that, in the step (X), a retention zone containing a first specific binding substance labeled with a labeling material, is provided between the sample application zone and the detection zone.

Also, in the step (d), the signal intensity may be continuously measured in the detection zone, along the permeating direction of the sample on the strip.

Further, in the step (d), the signal intensity may be determined from an analytical line showing the relation between a position in the permeating direction and the signal intensity.

The present invention further provides a specific binding analysis apparatus for quantitatively determining an analyte in a sample from a signal attributed to a specific binding reaction between the analyte and a specific binding substance capable of specifically binding to the analyte, characterized by comprising:
(1) a storage unit comprising a database which includes, for an individual sample containing a suspected analyte,
   at least the relation between a concentration of the analyte and a saturation value or change of a signal intensity attributed to a specific binding reaction between the analyte and a specific binding substance capable of specifically binding to the analyte;
   the change over time of the signal intensity; and
   time tₛ at which the saturation value is obtained and time tₘ at which a maximum value of the signal intensity is obtained, each of which being measured from the start of the specific binding reaction;
(2) a strip comprising a sample application zone where a sample containing an analyte is applied and a detection zone where a specific binding substance is practically immobilized and a signal attributed to a specific binding reaction can be detected;
(3) a first detector which detects the signal;
(4) a control unit which determines, based on the database, a measurement pattern of the signal intensity and causes the detector to detect the signal according to the measurement pattern, thereby determining an intensity of the signal; and
(5) an analysis unit which determines, based on the database, an amount of the analyte in the sample by using the signal intensity.

It is preferable that the above-described specific binding analysis apparatus further comprises a second detector which detects application of a sample to the sample application zone.

### Brief Description of Drawings

FIG. 1 is a flow chart of a specific binding analysis method in accordance with the present invention.
FIG. 2 is a graph showing the change over time of a signal intensity attributed to a specific binding reaction employing a sample at a particular concentration.
FIG. 3 is a graph showing the relation between a saturation value of a signal intensity and a concentration of an analyte.
FIG. 4 is a graph showing the change over time of a signal intensity attributed to a specific binding reaction employing a sample at a particular concentration.
FIG. 5 is a graph showing the relation between the rate of change over time of a signal intensity and a concentration of an analyte.
FIG. 6 is a schematic oblique view of an example of a strip used in the present invention.
FIG. 7 is a diagram conceptually illustrating the structure of an example of a specific binding analysis apparatus in accordance with the present invention.
FIG. 8 is an analytical line showing the relation (state of distribution) between a position in the permeating direction in the vicinity of a detection zone and the signal intensity.
FIG. 9 is a graph showing the change over time of a signal intensity attributed to a specific binding reaction employing a sample at a particular concentration.
FIG. 10 is a graph showing the relation between a saturation value of a signal intensity and a concentration of hCG.

### Best Mode for Carrying Out the Invention

### A. Specific binding analysis method

The present invention relates to a specific binding analysis method of quantitatively determining an analyte in a sample from a signal attributed to a specific binding reaction between the analyte in the sample and a specific binding substance capable of specifically binding to the analyte, characterized by comprising the steps of:
(a) previously preparing a database comprising, for an individual sample containing a suspected analyte,
   at least the relation between a concentration of the analyte and a saturation value or change of a signal intensity attributed to a specific binding reaction between the analyte in the sample and a specific binding substance capable of specifically binding to the analyte,
   the change over time of the signal intensity, and
   time tₛ at which the saturation value is obtained and time tₘ at which a maximum value of the signal intensity is obtained;
(b) preparing a sample containing an analyte and determining, based on the database, a measurement pattern of the signal intensity corresponding to the sample,
(c) causing a specific binding reaction between the analyte and a specific binding substance,
(d) measuring, based on the measurement pattern, the signal intensity at least twice in a period in which a signal intensity attributed to the specific binding reaction reaches saturation, after the step (c); and
(e) determining, based on the database, an amount of the analyte in the sample by using at least two signal intensities obtained in the step (d).

In the present invention, when the amount of an analyte contained in a sample is measured by detecting a signal exhibited by the analyte in a specific binding reaction, a database containing a signal attributed to the specific binding reaction and the like is previously produced for various samples, in order to solve the above-described problems of the prozone phenomenon and background. Then, when a quantitative determination of a sample is carried out, a measurement pattern of the signal is determined based on the database according to the type and the like of the sample to actually measure a signal, and then the amount of the analyte is determined based on the measured value of the signal and on the database.

Herein, the sample used in the present invention is a liquid sample suspected of containing an analyte. Examples include, urine, blood serum, blood plasma, whole blood, saliva, lacrimal fluid, spinal fluid and secretion from papillae. The sample may also be prepared by suspending or dissolving, in a liquid such as a buffer solution, extract solution or dissolved solution, a solid substance, a gel substance or sol substance of mucus, human body tissue, cell or the like.

The analyte used in the present invention may be any one, which has a specific binding substance thereto capable of specifically binding to the analyte. Examples include various proteins, polypeptides, glycoproteins, polysaccharides, complex glycolipids, nucleic acids, effector molecules, receptor molecules, enzymes and inhibitors, each of which functions as an antibody or antigen. More specific examples include: tumor markers such as α-fetoprotein, carcinoembryonic antigen (CEA), CA 125 and CA 19-9; various proteins, glycoproteins or complex glycolipids such as β2-microglobulin (β2m) and ferritin; various hormones such as estradiol (E2), estriol (E3), human chorionic gonadotropin (hCG), luteinizing hormone (LH) and human placental lactogen (hPL); various virus-associated antigens or virus-associated antibodies such as HBs antigen, HBs antibody, HBc antigen, HBc antibody, HCV antibody and HIV antibody; various allergens and IgE antibodies thereto; narcotic drugs, medical drugs and metabolites thereof; and virus-associated and tumor-associated nucleic acids having a polynucleotide sequence.

The specific binding substance used in the present invention may be any substance which specifically binds to the above-described analytes, and examples include antibodies, antigens, glycoproteins, polysaccharides, complex glycolipids, nucleic acids, effector molecules, receptor molecules, enzymes and inhibitors.

In the present invention, it is preferable to use a first specific binding substance labeled with a labeling material as well as a second specific binding substance, and to cause the labeled first specific binding substance to bind, via an analyte, to the second specific binding substance. The reason is that, according to such a method, the method of the present invention can be suitably performed, for example, by previously immobilizing the second specific binding substance at a given site, and then measuring and detecting a signal attributed to the labeling material only at the site containing the immobilized substance.

Additionally, in terms of high specificity, it is preferable that at least one of the first specific binding substance and the second specific binding substance is an antibody. Further, it is preferable to use a monoclonal antibody.

The first specific binding substance and second specific binding substance to be used may not necessarily be same substances. In addition, when the analyte does not have a plurality of same epitopes, it is preferable to use substances having different specificities against respective different epitopes, as the first specific binding substance and second specific binding substance. Of course, when the analyte has a plurality of same epitopes, same specific binding substances may be used as the first specific binding substance and second specific binding substance. It should be noted that the analyte itself may be labeled with a labeling material, instead of using a labeled first specific binding substance.

A substance or a labeled substance which exhibits the same behavior as that of the analyte in the specific binding reaction with the second specific binding substance, may be made to coexist with the analyte. The reason is that this enables a quantitative determination which utilizes competitive reactions, as well as increasing the expectation that the prozone will be avoided.

In the case of binding the analyte to the first specific binding substance outside the strip, it is preferable to use an unlabeled first specific binding substance and then to cause another labeled specific binding substance to react with the analyte. This is because, by previously causing the analyte to react with the unlabeled specific binding substance, the amount of the analyte bound to the labeled specific binding substance can be reduced, thereby enabling a quantitative determination which utilizes competitive reactions, as well as increasing the expectation that the prozone will be avoided.

The first specific binding substance to be used may also be a substance bound to a labeling material which generates a different signal from that of the labeling material of the specific binding substance retained in the matrix. In this case, it is possible to select which signal to be read at a detection zone 5, thereby enabling a quantitative determination which utilizes competitive reactions, as well as increasing the expectation that the prozone will be avoided.

The labeling material used in the present invention may be any substance whose presence can be freely detected. Examples include: a direct label such as a labeling material visible by naked eyes in the natural state, a labeling material visible with the use of an optical filter or a labeling material visible when stimulated with an ultraviolet ray or the like to promote its fluorescence; and an indirect label such as a labeling material whose visible signal is detectable by adding therewith a developing reagent such as a substrate.

Examples of the direct label include minute colored particles such as dye sols, metal sols or colored latex particles, and particles containing a fluorescent substance. On the other hand, examples of the indirect label include enzymes such as alkaline phosphatase and horseradish peroxidase. The direct label may be preferably used, since it generates a signal detectable without addition of a different reagent and thereby instantaneously provides an analytical result, as well as being durable and stable. Particularly, the use of colored particles of colloidal gold or the like allows a labeled portion to be concentrated in a small zone or volume, because the colored particles are minute.

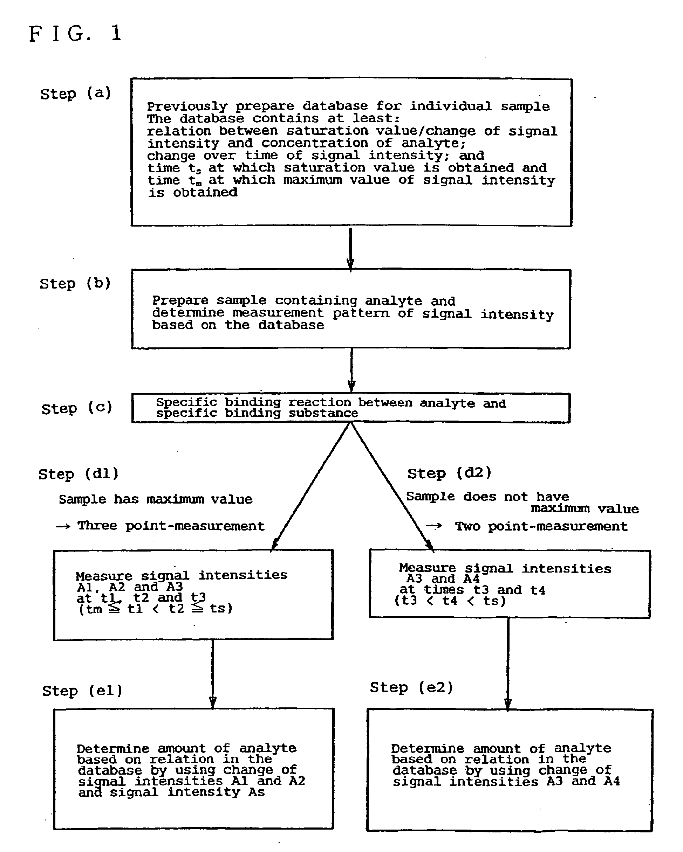

Herein, in order to facilitate understanding of the present invention, the present invention will be described with reference to the flow chart shown in FIG. 1. FIG. 1 is a flow chart of a specific binding analysis method in accordance with the present invention.

Firstly, in step (a), for an individual sample containing a suspected analyte, a database is previously prepared, which comprises: at least the relation between a concentration of the analyte and a saturation value or change of a signal intensity attributed to a specific binding reaction between the analyte in the sample and a specific binding substance capable of specifically binding to the analyte; the change over time of the signal intensity; and time tₛ at which the saturation value is obtained and time tₘ at which a maximum value of the signal intensity is obtained.

Herein, the saturation value refers to a signal intensity attributed to a specific binding reaction between the analyte and the specific binding substance, which is obtained after the specific binding reaction has reached equilibrium. Because the specific binding reaction gradually proceeds after reactants are contacted with each other, the obtained signal intensity gradually changes if the signal intensity is measured continuously. Further, since the specific binding reaction is an equilibrium reaction, almost no change is seen in the signal intensity after equilibrium has been finally reached. In other words, a saturation value of the signal intensity is a terminal value of the signal intensity in an equilibrium state.

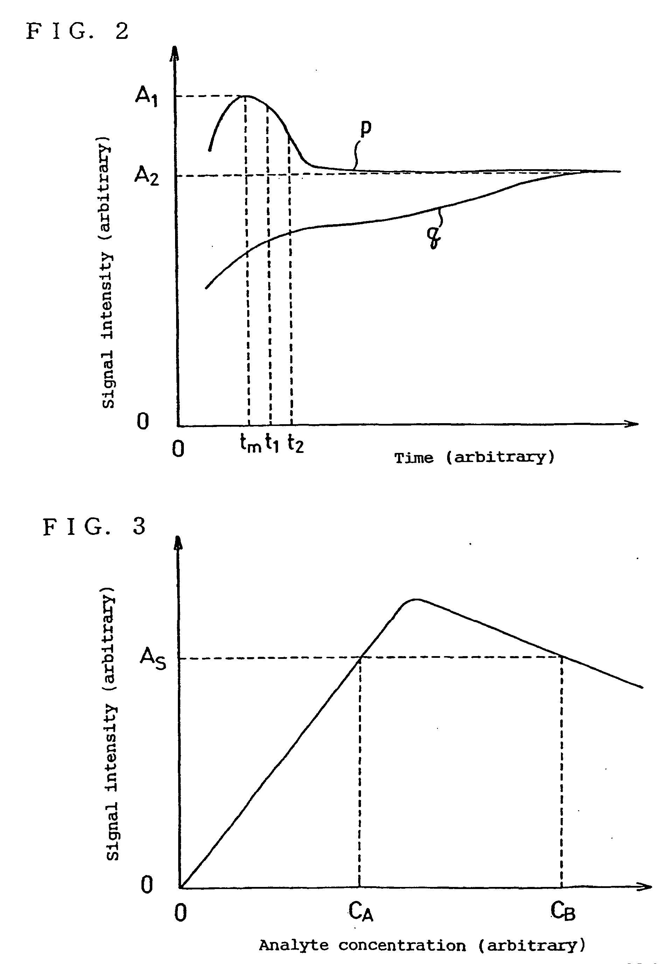

Information for a quantitative determination contained in the above-described database may be represented, for example, by the graphs shown in FIGS. 2 to 5.

For example, when the amount of an analyte in a sample is excessive, a signal intensity attributed to the analyte initially increases in proportion to time (as linear function); however, the signal intensity gradually decreases owing to the remaining analyte which has not bound to the specific binding substance (prozone phenomenon). Therefore, a graph illustrating the change over time of a signal intensity attributed to a specific binding reaction is prepared as shown in FIG. 2, by employing Sample "p" at a particular concentration. It should be noted that, as in the case of Sample "q", some samples do not produce the prozone phenomenon but eventually exhibit the same signal intensity as that of Sample "p". The graph shown in FIG. 2 includes time tₛ at which the saturation value is obtained and time tₘ at which a maximum value of the signal intensity is obtained. Since FIG. 2 shows the time at which a saturation value of the signal intensity is obtained, a graph showing the relation between the concentration of the analyte in the sample and a saturation value of the signal intensity, is prepared next (FIG. 3). FIG. 3 also shows that the sample containing an analyte at a high concentration produces the prozone phenomenon.

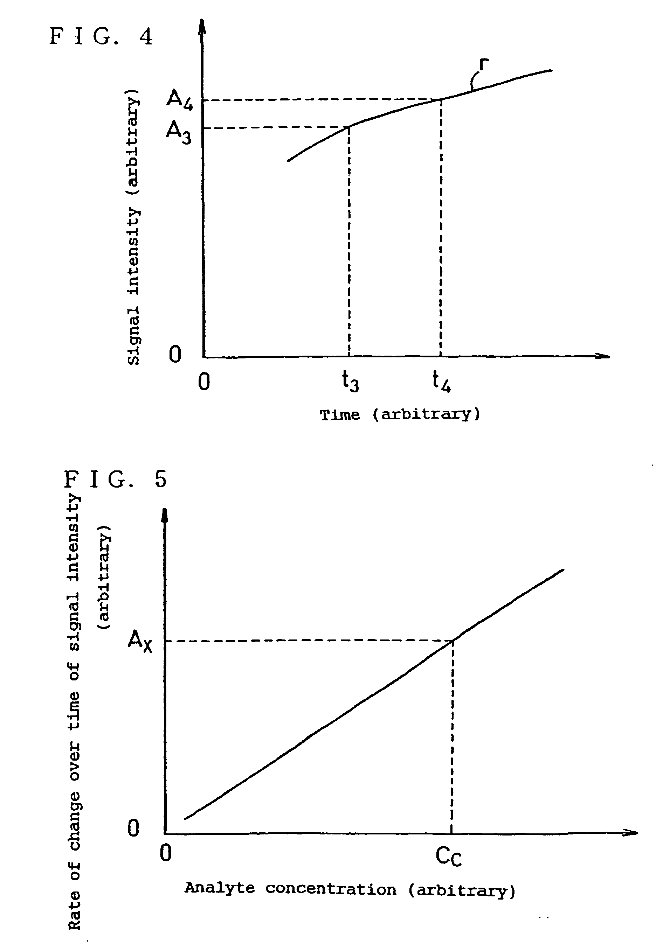

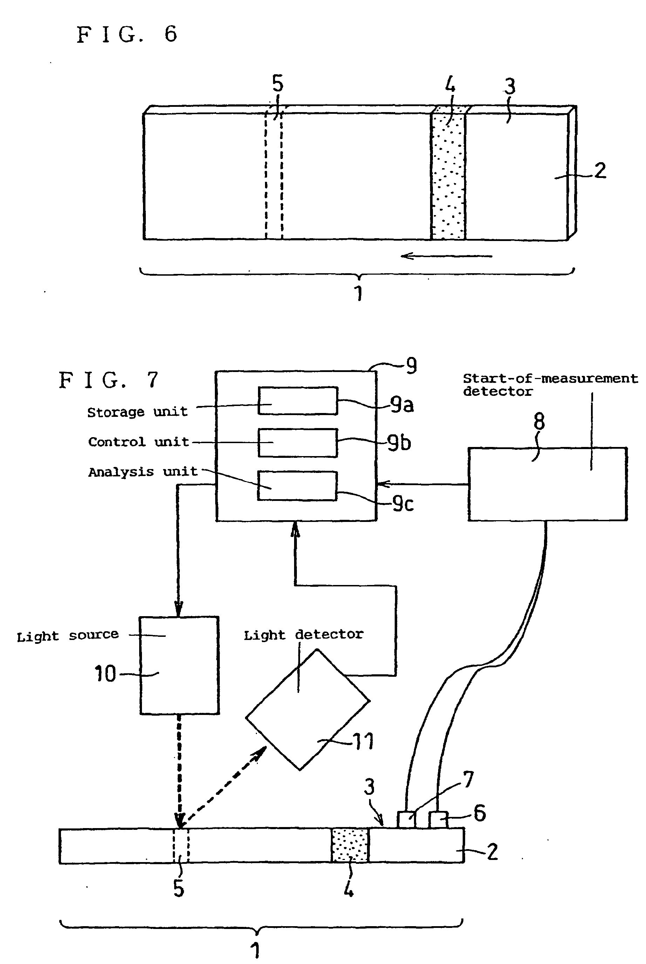

On the other hand, for example, when the amount of an analyte in a sample is small, a graph showing the change over time of a signal intensity attributed to a specific binding reaction is prepared as shown in FIG. 4, by employing Sample "r" at a particular concentration. Although this graph contains time tₛ at which the saturation value is obtained, it does not give a maximum value of the signal intensity. Therefore, the above-described database also contains the information that the maximum value is not present. Then, since FIG. 4 shows that a signal intensity attributed to the analyte increases in proportion to the concentration of the analyte, a graph showing the relation between the rate of change over time of the signal intensity and the concentration of the analyte, is prepared as shown in FIG. 5.

It should be noted that although the signal intensities measured by continuously monitoring the signal intensity were shown as the analytical line in FIGS. 2 to 5. the analytical line may also be obtained by measuring the signal intensity at a plurality of time points and connecting the respective measurement points with lines.

By previously preparing the database in the above-described manner, when a sample is used for a specific binding measurement, a possible behavior and the like of the sample can be known in advance, so that the above-described influences of the prozone phenomenon and background can be avoided with a minimum number of measurements by using the measurement pattern according to such behavior. In particular, in the case of a sample at a high concentration, erroneous measurement caused by the prozone phenomenon can be avoided by using a first measurement pattern described below. On the other hand, in the case of a sample at a low concentration, the background gradually disappears if a long time has elapsed from the start of the specific binding reaction, but this necessitates a long waiting time; accordingly, a second measurement pattern described below is used to enable a more rapid and accurate measurement.

Therefore, in the subsequent step (b), a sample containing an analyte is actually prepared, and a suitable measurement pattern of the signal intensity corresponding to the sample is determined based on the database.

Then, the analyte is subjected to a specific binding reaction with a specific binding substance (step (c)), and a signal intensity attributed to the specific binding reaction is measured based on the determined measurement pattern at least twice in a time period in which the signal intensity reaches saturation (step (d)). Subsequently, the amount (concentration) of the analyte in the sample corresponding to a saturation value of the signal intensity is determined based on the database, using at least two signals obtained in the step (d) (step (e)).

Herein, as described above, the measurement pattern is classified mainly into two types in the present invention. The first measurement pattern is used for measurement of a sample having the potential of producing the prozone phenomenon, and the second measurement pattern is used for measurement of a sample not having the potential of producing the prozone phenomenon. The presence or absence of the prozone phenomenon corresponds to the presence or absence of the maximum value in the database.

### (i) First measurement pattern

According to the first measurement pattern, when the sample in the step (b) has the maximum value judging from the database and produces the prozone phenomenon, signal intensities A₁ and A₂ are measured at least at times t₁ and t₂ (tₘ ≦ t₁ < t₂ ≤ tₛ), respectively, and saturation value Aₛ of the signal intensity is measured at the time tₛ; then, in the step (e), the amount of an analyte in the sample is determined by using the change of the signal intensities A₁ and A₂ (e.g., rate of change, difference or whether these are positive or negative) as well as a saturation value of Aₛ of the signal intensity, based on the relation (e.g., FIG. 2) in the database.

More specifically, FIG. 3 shows the two different concentrations C_{A} and C_{B} can be considered as the analyte concentration corresponding to the saturation value Aₛ of the signal intensity. As shown by the analytical line of Sample "p" in FIG. 2, it is confirmed that the prozone phenomenon is produced when the rate of change {(A₂ - A₁)/(t₂ - t₁)} and difference (A₂ - A₁) of the signal intensity are negative; accordingly, the concentration of the analyte can be determined as C_{B} from the saturation value Aₛ of the signal intensity and FIG. 3.

In the event that the sample has the same saturation value Aₛ of the signal intensity as that of the analytical line of Sample "p" and does not produce the prozone phenomenon, as shown in the analytical line of Sample "q" in FIG. 2, the rate of change of the signal intensity {(A₂- A₁)/(t₂ - t₁)} and difference (A₂ - A₁) become positive and the production of the prozone phenomenon is not recognized; accordingly, the concentration of the analyte can be determined as C_{A} from FIG. 3.

The first measurement pattern will be described in further detail. As described above, when an excessive amount of an analyte is present in a sample and the prozone phenomenon is produced, there is a certain period of time during which the change of the signal intensity is steadily negative. Therefore, it is possible to judge whether or not the prozone phenomenon is produced by examining whether the change of the signal intensity is positive or negative (direction of change). Whether the change of the signal intensity is positive or negative can be examined by determining the rate of change and difference of the signal intensity.

From signal intensities A₁ and A₂, which were measured at least twice,at different times, for example, measurement times t₁ and t₂, the difference of the signal intensity can be determined by (A₂ - A₁). Similarly, from signal intensities A₁ and A₂, which were measured at least twice at different times, for example, measurement times t₁ and t₂ (tₘ ≤ t₁ < t₂ ≤ tₛ), the rate of change of the signal intensity can be determined by {(A₂ - A₁)/(t₂ - t₁)}. The rate of change of the signal intensity can also be determined by employing linear regression using the least-squares method.

Accordingly, in the first measurement pattern, the signal intensity is measured at least twice in order to examine whether the change of the signal intensity is positive or negative. When there are plural concentrations which are corresponded to a saturation value of the measured signal intensity from the database (FIG. 3) and the change of the signal intensity is positive, it is judged that there is no time period during which the signal intensity is steadily negative, i.e., the prozone phenomenon is not produced; accordingly, of the plural concentrations, a lower concentration is determined as the concentration of the analyte. On the other hand, when the change of the signal intensity is negative, it is judged that there is a time period during which the change of the signal intensity is steadily negative, i.e., that the prozone phenomenon is produced; accordingly, of the plural concentrations, a higher concentration is determined as the concentration of the analyte.

Additionally, when judging whether or not the prozone phenomenon is produced by using the direction of change of the signal intensity, it is necessary to measure the signal intensity at a time period during which the signal intensity is steadily low. Therefore, it is desirable to judge the change of the signal intensity from the intensity of at least two signals measured at times t₁ and t₂(tₘ ≦ t₁ < t₂ ≦ tₛ), i.e., from the time when the signal intensity has reached a maximum value to the time when it has reached a saturation value.

In other words, in the first measurement pattern, it is necessary to measure the signal intensity at least three times in total: at least once for obtaining a saturation value of the signal intensity and at least twice for obtaining the change of the signal intensity, according to the elapsed time measured by a timer or the like. For example, the database shown in FIG. 2 indicates that the signal intensity steadily decreases in a time period between the application of the sample and 2 to 3 minutes thereafter, so that the signal intensity is measured at least twice in this time period in order to obtain the change of the signal intensity. Then, in order to obtain the saturation value, the signal intensity is measured at least once after the specific binding reaction between the analyte and the second specific binding substance has reached saturation. In addition, since the measurement time point varies depending on the analyte, specific binding substance and the like, it is preferable to prepare a large number of databases corresponding to such requirements.

### (ii) Second measurement pattern

On the other hand, according to the second measurement pattern, when the sample in the step (b) does not have the maximum value judging from the database and does not produce the prozone phenomenon, signal intensities A₃ and A₄ are measured at least at times t₃ and t₄ (t₃ < t₄ < tₛ), and, in the step (e), the amount of an analyte in the sample is determined based on the change (rate of change or difference) of the signal intensities A₃ and A4, and the relation (e.g., FIG. 5) in the database.

Specifically, by previously preparing a graph showing the relation between the rate of change over time of the signal intensity and the concentration of the analyte, as shown in FIG. 5, the concentration of the analyte can be determined as C_{c} from FIG. 5 by calculating the rate of change over time of the signal intensity Aₓ (= {(A₄ - A₃)/(t₄ - t₃)}) shown by the analytical line of Sample "r" in FIG. 4.

The second measurement pattern will be described in further detail. When an excessive amount of an analyte is not present in a sample, particularly when the concentration of an analyte in a sample is low, the signal intensity simply increases with time until it reaches a saturation value. Therefore, it is possible to measure the change of the signal intensity (e.g., difference or rate) by measuring the signal intensity in an earlier time period before it reaches a saturation value. On the contrary, since the background of the signal intensity may disappear with the passage of time, it is difficult to subtract the signal intensity attributed to the background from the signal intensity measured just before or after the disappearance of the background. Moreover, a long time is required to wait for the background to disappear. Therefore, in the second measurement pattern, the signal intensity is measured at least twice, regardless of whether or not the background disappears.

In other words, when calculating the concentration of the analyte using the change of the signal intensity, it is preferable to complete the measurement in a period from the time when the specific binding reaction has been practically terminated to the time when the signal intensity has reached a saturation value. The obtained signal intensity is influenced by the foreign matter and background as well as by the specific binding reaction between the analyte and the specific binding substance. Therefore, the difference of the signal intensity measured in an earlier time period after the start of the specific binding reaction corresponds to the difference of the signal intensity attributed to the specific binding reaction between the analyte and the specific binding substance; accordingly, it is possible to cancel the influences of the background and foreign matter.

As in the first measurement pattern, from signal intensities A₃ and A₄, which were measured at least twice at different times, for example, measurement times t₃ and t₄ (t₃ < t₄ < tₛ), the difference of the signal intensity can be determined by (A₄ - A₃). Similarly, from signal intensities A₃ and A₄, which were measured at least twice at different times, for example, measurement times t₃ and t₄ (tₘ ≦ t₁ < t₂ ≦ tₛ), the rate of change of the signal intensity can be determined by {(A₄ - A₃)/(t₄ - t₃)}. The rate of change of the signal intensity can also be determined by employing linear regression using the least-squares method.

The specific binding analysis method of the present invention as described above can be performed using a test reagent strip having a particular structure.

Therefore, it is preferable that the specific binding analysis method of the present invention further comprises,
prior to the step (b), step (X) of producing a strip comprising a sample application zone where the sample is applied and a detection zone where a second specific binding substance is substantially immobilized and a signal attributed to a specific binding reaction can be detected,
wherein, in the step (d), the sample is applied to the sample application zone to allow the sample to flow into the detection zone by capillarity, thereby causing the analyte bound with the first specific binding substance to specifically bind to the second specific binding substance.

Herein, the above-mentioned strip will be described with reference to a drawing. FIG. 6 is a schematic oblique view of an example of a strip used in the present invention. A strip 1 used in the present invention is, for example, a sheet-like test strip comprising a matrix 2 capable of causing capillarity, and a liquid sample, when applied on one end of the strip, permeates and develops by capillarity to the other end in the direction shown by the arrow.

The strip 1 has a sample application zone 3, a retention zone 4 and a detection zone 5. The sample application zone 3 is a region where the sample is applied on the matrix 2. The retention zone 4, which is provided between the sample application zone 3 and the detection zone 5, is a region where the applied sample flows in, and contains a first specific binding substance labeled with a labeling material. However, it is not necessary to form the retention zone 4 in the case of using a previously labeled sample. The detection zone 5 is a region where the sample flows in via the retention zone 4 and has a second specific binding substance immobilized therein as a binding material.

Accordingly, any material capable of forming a site where an analyte and a specific binding substance are developed, may be used as the material constituting the above-described matrix, and examples include a porous carrier, a gel carrier and a fine particle-packed carrier.

In particular, nitrocellulose may preferably be used. Nitrocellulose is superior to other matrix materials such as paper, because it is inherently capable of binding to protein without being previously sensitized. When directly applied to nitrocellulose, a specific binding substance such as an antibody can be reliably immobilized, without requiring any chemical treatment which might hinder the specific binding capability of the specific binding substance. When the matrix material is, for example, paper, the immobilization of an antibody necessitates a chemical binding to be performed using CNBr, carbonyldiimidazole, tressil chloride or the like. Moreover, nitrocellulose sheets are commercially available in various pore sizes, so that the matrix material can be readily selected according to requirements such as the flow rate of sample. In the case of using a nitrocellulose sheet, it is preferable to use a composite sheet comprising a nitrocellulose sheet and a backing sheet, such as a plastic sheet, attached on the back of the nitrocellulose sheet, from the viewpoints of strength and handleability. Such a composite sheet may readily be produced, for example, by forming a thin film of nitrocellulose on a backing sheet.

In order to retain the first specific binding substance in the retention zone 4, for example, a liquid substance containing the first specific binding substance may be applied on a predetermined region on the matrix 2, followed by drying. Even if the retention zone 4 is in the dried state as described above, the retention zone 4 is wetted when the sample permeates and develops on the matrix 2 to flow into the retention zone 4, so that the first specific binding substance can freely migrate on the matrix 2.

After the second specific binding substance is immobilized in the detection zone 5, it is preferable to block the remaining portions except for the portion where the second specific binding substance is immobilized, thereby reducing nonspecific absorption to the matrix 2. The blocking may be performed by application of, for example, protein (e.g., bovine serum albumin and milk protein), polyvinylalcohol or ethanolamine, or a combination thereof.

Accordingly, when the sample is applied to the sample application zone 3 in the step (d), the sample flows into the detection zone 5 by capillarity, and then the analyte bound to the first specific binding substance specifically binds to the second specific binding substance retained in the detection zone 5.

In addition, the analyte in the sample may be specifically bound to the first specific binding substance prior to being applied to the sample application zone 3, and the sample may be thereafter applied to the sample application zone 3, as described above. The first specific binding substance to be reacted with the analyte in the sample outside the strip 1 may not be bound to a labeling material. When the first specific binding substance labeled with a labeling material is contained in the retention zone 4 in the matrix 2. the first specific binding substance bound to a labeling material which generates a signal different from that of the above-mentioned labeling material, may be used outside the strip. Further, when the labeled first specific binding substance is previously reacted with the sample outside the strip, it is not necessary to provide the retention zone 4 containing the first specific binding substance.

The analyte in the sample that has reached the detection zone 5 specifically binds to the second specific binding substance. As a result, the analyte is immobilized in the detection zone 5, via the second specific binding substance. For example, a colloidal gold labeled anti-hCG monoclonal antibody as the first specific binding substance binds, via an hCG as the analyte, to an anti-hCG monoclonal antibody as the second specific binding substance immobilized in the detection zone 5.

Herein, it is preferable that the sample is developed such that the sample keeps flowing even after it migrates beyond the detection zone 5. For this purpose, a sufficient amount of the sample is applied to the sample application zone 3 so that a surplus of the labeled first specific binding substance, which does not participate in the binding reaction, for example, is allowed to migrate beyond the detection zone 5 to be washed off by the sample itself from the detection zone 5. Therefore, an absorption zone where the sample flowed out from the detection zone 5 is absorbed may be provided on the strip 1 at the end of the matrix 2 where the sample is developed. The material constituting the absorption zone may be any material having an absorptive property to sufficiently wash off the sample other than the analyte from the detection zone 5. Examples include the glass fiber filter paper GA-200 (manufactured by TOYO KABUSHIKI KAISHA). With the provision of the absorption zone, any unreacted substance can be washed off together with the flow of the sample, so that after the specific binding reaction, a signal attributed to the specific binding reaction can be detected in the detection zone 5 without performing separation of the unreacted substance.

Accordingly, in the specific binding analysis method of the present invention, it is possible to measure a signal intensity attributed to a specific binding reaction between an analyte and a specific binding substance, at any portion in a site where the specific binding reaction occurs. However, from the viewpoint of accuracy, it is preferable to quantitatively or qualitatively determine the amount of the analyte in the sample by measuring the signal intensity in the detection zone 5 or in a wide region including the detection zone 5.

Then, by detecting the distribution of the signal intensity in a region including the detection zone 5 after the specific binding reaction between the analyte and a second specific binding substance has reached equilibrium, the amount of the analyte in the sample can be quantitatively or qualitatively determined from the distribution of the signal intensity.

### B. Specific binding analysis apparatus

Next, descriptions will be made on a specific binding analysis apparatus which can be used for the above-described specific binding analysis method of the present invention. With the use of the specific binding analysis apparatus, the above-described specific binding analysis method can be more suitably performed.

The present invention relates to a specific binding analysis apparatus for quantitatively determining an analyte in a sample from a signal attributed to a specific binding reaction between the analyte and a specific binding substance capable of specifically binding to the analyte, characterized by comprising:
(1) a storage unit comprising a database which includes, for an individual sample containing a suspected analyte,
   at least the relation between a concentration of the analyte and a saturation value or change of a signal intensity attributed to a specific binding reaction between the analyte and a specific binding substance capable of specifically binding to the analyte;
   the change over time of the signal intensity; and
   time tₛ at which the saturation value is obtained and time tₘ at which a maximum value of the signal intensity is obtained, each of which being measured from the start of the specific binding reaction;
(2) a strip comprising a sample application zone where a sample containing an analyte is applied and a detection zone where a specific binding substance is practically immobilized and a signal attributed to a specific binding reaction can be detected;
(3) a first detector which detects the signal;
(4) a control unit which determines, based on the database, a measurement pattern of the signal intensity and causes the detector to detect the signal according to the measurement pattern, thereby determining an intensity of the signal; and
(5) an analysis unit which determines, based on the database, the amount of the analyte in the sample by using the signal intensity.

Further, it is preferable that the specific binding analysis apparatus further comprises a second detector which detects application of a sample to the sample application zone.

Herein, the specific binding analysis apparatus of the present invention will be described with reference to a drawing. FIG. 7 is a diagram conceptually illustrating the structure of an example of the specific binding analysis apparatus of the present invention.

The specific binding analysis apparatus shown in FIG. 7 comprises: a light source 10; a light detector 11 as a first detector; a computer 9 comprising a storage unit 9a, a control unit 9b and an analysis unit 9c; and a strip 1. A start-of-measurement detector 8 which detects the application of a sample to the application zone 3 is provided as a second detector.

The storage unit 9a in the computer 9 contains, for an individual sample containing a suspected analyte, at least the relation between the concentration of the analyte and a saturation value or change of a signal intensity, the intensity being attributed to a specific binding reaction between the analyte and a specific binding substance capable of specifically binding to the analyte, and the change over time of the signal intensity. The storage unit 9a also contains time tₛ at which the saturation value is obtained and time tₘ at which a maximum value of the signal intensity is obtained, with a predetermined time point, such as the time point of the start of the specific binding reaction or the time point of the application of the sample, taken as zero.

In the control unit 9b, the computer 9 then determines a measurement pattern of the signal intensity according to the type or the like of the sample based on the database, and sends the light detector 11 an instruction to perform detection when a predetermined time has elapsed from the start of the specific binding reaction or that of the detection of the signal, based on the determined measurement pattern. Then, the light detector 11 recognizes the intensity of the detected signal. The analysis unit 9c then uses the recognized signal intensity to determine the amount of the analyte in the sample based on the database. Therefore, the computer 9 has a timer function.

The light source 10 applies light to the detection zone 5, whereupon the light detector 11 detects a reflected light from the detection zone 5. The light source 10 may apply light to the vicinity of the detection zone 5, either constantly during the measurement or only at a time necessary for the measurement of the signal intensity.

Further, the start-of-measurement detector 8, which has a first electrode 6 and a second electrode 7, measures an electrical conductivity of the application zone 3 to detect the application of a sample to the sample application zone 3 based on the change of the electrical conductivity. Prior to the application of a sample, an electrical conductivity of the sample application zone 3 in the dry state and an electrical conductivity of the sample application zone 3 in the wet state after the application of a sample are previously measured, and the obtained measured values are stored as a start-of-measurement information in the start-of-measurement detector 8. Thereafter, when the sample application zone 3 turns from dry to wet upon the application of a sample containing an analyte to the sample application zone 3, the electrical conductivity of the sample application zone 3, which is monitored by the first electrode 6 and second electrode 7, changes; then, the start-of-measurement detector 8 detects the application of the sample to the sample application zone 3 by making reference to the change of the electrical conductivity and to the start-of-measurement information.

Also, the storage unit 9a in the computer 9 may store, as the start-of-measurement information, an electrical conductivity in the dry state and an electrical conductivity in the wet state, and the analysis unit 9b may cause the control unit 9c to start the measurement by making reference to an actual change of the electrical conductivity and the start-of-measurement information.

In the following, the method of operating the specific binding analysis apparatus of the present invention will be described in further detail. It is preferable to apply the sample to the application zone 3 after the strip 1 is placed in the specific binding analysis apparatus, but alternatively, the strip 1 applied with a sample may be placed in the specific binding analysis apparatus.

When the start-of-measurement detector 8 detects the application of a sample, the computer 9 makes a timer (not shown) to start measuring time, with the time point of the application of the sample taken as zero, while controlling the light source 10 and the light detector 11 to measure the signal intensity at a predetermined time interval.

The light source 10 applies light of a predetermined wavelength (e.g., 520 nm) to a region including the detection zone 5, whereupon the light detector 11 detects the reflected light. As the wavelength used for the measurement, any wavelength suitable for the coloration of a sample or that of a labeling material in the detection zone 5, may be appropriately selected.

The signal as used herein may be any detectable signal that can be generated by a reaction in which a labeling material participates, for example: fluorescence measurable with a fluorometer; luminescence measurable with a luminescence photometer; and coloration measurable with a visual evaluation or with a color-difference meter in the detection zone 5. In this case, the intensity of the like of reflected light, fluorescence or luminescence is detected in the detection zone 5.

The detection of the above-described signal may be continuously performed, while relatively changing the position of the strip 1 and the positions of the light source 10 and light detector 11 in the direction parallel to the permeating (proceeding) direction of the sample on the strip 1. Alternatively, either one of the strip 1 and the light source 10 may be moved, or both of them may be moved together, in the direction parallel to the permeating direction of the sample.

The light detector 11 sends a detected signal to the computer 9. The analysis unit 9c in the computer 9 analyzes the signal from the light detector 11, thereby analyzing the intensity of the signal. The analysis unit 9c analyzes a saturation value, rate of change and the like of the signal intensity in the database stored in the storage unit 9a, based on the analyzed signal intensity.

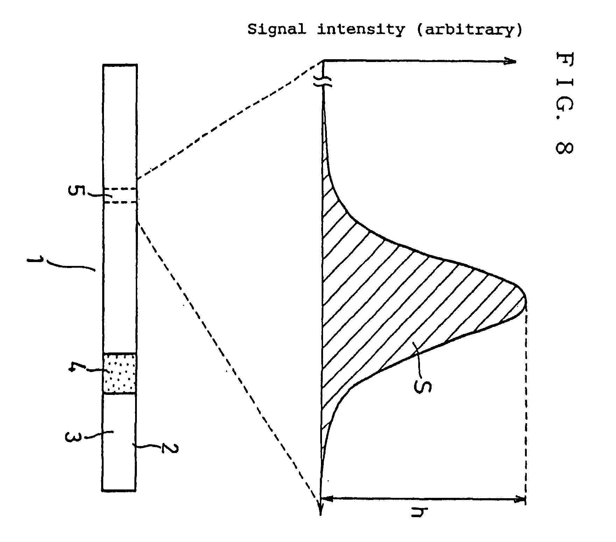

Herein, FIG. 8 shows an analytical line showing the relation (state of distribution) between a position in the permeating direction in the vicinity of the detection zone and the signal intensity. This analytical line was prepared by applying light on the matrix 2 from the light source 10 while scanning the strip 1 or the light source 10, and analyzing a reflected light detected by the light detector 11 with the computer 9. In this analytical line, the vertical axis denotes the signal intensity in the vicinity of the detection zone 5, and the horizontal axis denotes the position on the strip 1 in the permeating direction of the sample (the distance from the sample application zone 3). The height "h" of this analytical line can be measured as the signal intensity attributed to a specific binding reaction. Also, the area "s" of this analytical line may be considered as the signal intensity attributed to a specific binding reaction.

It should be noted that in the embodiments of the present invention described below, the signal intensity is obtained by continuously measuring a reflected light in a region including the detection zone 5, while scanning the strip 1 or the light source 10. Then, for example, a signal intensity in an upstream portion, which is close to the sample application zone 3, of the detection zone 5, is connected with line to a signal intensity in the downstream portion thereof, i.e., the opposite side, in the above-described analytical line. As a result, the height "h" obtained by subtracting the height of the straight line from that of the highest value in the detection zone 5 and the area "S" surrounded by the analytical line and the straight line, corresponds to a signal intensity attributed to a specific binding reaction, which is obtained by subtracting the signal intensity attributed to the background from the signal intensity of the detection zone 5. By measuring the signal intensity in this manner, it is possible to quantitatively or qualitatively determine the amount of an analyte even in a colored sample such as whole blood, without being influenced by the background and foreign matter.

Also, a signal intensity on the matrix 2 in a given portion other than the detection zone 5 may be subtracted from the measured signal intensity, assuming that this signal intensity is a signal intensity attributed to the background. As the signal intensity attributed to the background, for example, one with the smallest value may be selected from signal intensities in the vicinity of the detection zone 5.

In addition to the above-described methods, compact and simplified structure, cost reduction as well as wide spread use of the measurement apparatus can be achieved by, for example, immobilizing a site where a signal intensity is measured, such as the detection zone 5, and measuring the signal intensity without moving the site where a specific binding reaction occurs or the measurement apparatus. In the following, the present invention will be described in further detail with reference to examples; however, the present invention is not limited thereto.

### Example 1

In this example, urine was used as a sample and human chorionic gonadotropin (hCG) was used as an analyte contained in the sample. In addition, an anti-hCG monoclonal antibody, capable of participating in a sandwich reaction with an hCG, was used as a first specific binding substance as well as a second specific binding substance, and colloidal gold was used as a labeling material. The colloidal gold as the labeling material was bound to the first specific binding substance bound via the analyte to the second specific binding substance, so that it was possible to quantitatively or qualitatively determine the hCG in the detection zone 5 in an accurate manner, by using a signal attributed to a reaction in which the colloidal gold participated.

### Step (a)

In order to produce a database by the step (a), a strip 1 with the structure shown in FIG. 6 containing a matrix 2 comprising nitrocellulose, was produced first. A colloidal gold labeled anti-hCG monoclonal antibody (a-hCG), which is the first specific binding substance and capable of immunologically binding to the hCG, was retained in a retention zone 4. More specifically, the a-hCG was applied to the matrix, followed by drying. Further, the a-hCG as the second specific binding substance was also applied to a detection zone 5, followed by drying to immobilize the a-hCG.

Six different urines having various hCG concentrations were previously prepared as samples each containing a suspected analyte. The hCG concentration was 100 (IU/L). 500 (IU/L), 1000 (IU/L), 1500 (IU/L), 2000 (IU/L) or 2500 (IU/L).

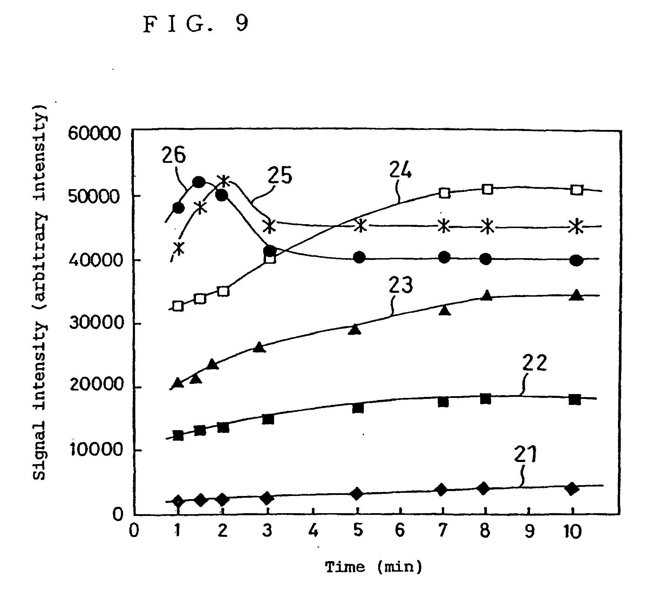

Then, with the use of the specific binding analysis apparatus shown in FIG. 7, the application of urine to a sample application zone 3 was detected at a start-of-measurement detector 8, and a signal intensity attributed to the specific binding reaction in the detection zone 5 was measured. For the six different urines, the change over time of the signal intensity, as well as time tₛ at which the saturation value was obtained and time tₘ at which a maximum value of the signal intensity was obtained, each of which was measured after the start of the specific binding reaction, were determined and they were shown in FIG. 9.

The signal intensity of the urine having an hCG concentration of 100 (IU/L) was denoted by "◆", and the change over time of the signal intensity was denoted by Line 21. The signal intensity of the urine having an hCG concentration of 500 (IU/L) was denoted by **"■",** and the change over time of the signal intensity was denoted by Line 22. The signal intensity of the urine having an hCG concentration of 1000 (IU/L) was denoted by **"▲",** and the change over time of the signal intensity was denoted by Line 23. The signal intensity of the urine having an hCG concentration of 1500 (IU/L) was denoted by "□", and the change over time of the signal intensity was denoted by Line 24. Also, the signal intensity of the urine having an hCG concentration of 2000 (IU/L) was denoted by "*", and the change over time of the signal intensity was denoted by Line 25. Further, the signal intensity of the urine having an hCG concentration of 2500 (IU/L) was denoted by **"●",** and the change over time of the signal intensity was denoted by Line 26.

In the cases of the urines having low hCG concentrations, the signal intensity increased with time, and subsequently reached saturation. The hCG bound to the labeled a-hCG in the retention zone 4 was gradually developed to the detection zone 5 with its signal intensity increasing with time, and thereafter, the specific binding reaction between the hCG and the a-hCG as the second specific binding substance reached equilibrium, whereupon the signal intensity reached saturation.

Line 21 showed that the specific binding reaction reached equilibrium after 7 minutes in the case of the urine having an hCG concentration of 100 (IU/L); accordingly, time tₛ at which a saturation value of the signal intensity was obtained, was 7 minute. Further, Lines 22, 23 and 24 showed that the specific binding reaction reached equilibrium after 8 minutes (tₛ = 8 min.) in the respective cases of the urines having hCG concentrations of 500 (IU/L), 1000 (IU/L) and 1500 (IU/L).

In contrast, in the cases of the urines having high hCG concentrations, the signal intensity increased with time, decreased after a certain period of time, and thereafter reached saturation. The reason for such a change of the signal intensity was presumably attributed to the fact that the hCG not bound to the labeled a-hCG was excessively present in the reaction system. In other words, in the cases of the urines having high hCG concentrations, the hCG bound to the labeled a-hCG in the retention zone 4 was gradually developed to the detection zone 5, so that the signal intensity initially increased with time. However, since the hCG not bound to the labeled a-hCG was excessively present in the reaction system, the hCG bound to the labeled a-hCG and the hCG not bound to the labeled a-hCG competed to specifically bind to the a-hCG immobilized in the detection zone 5, resulting in a decrease of the signal intensity detected in the detection zone 5, i.e., the signal intensity attributed to the reaction in which the labeling material participated. Thereafter, when the specific binding reaction between the hCG and the a-hCG as the second specific binding substance reached equilibrium, the signal intensity reached saturation.

Line 25 showed that in the case of the urine having an hCG concentration of 2000 (IU/L), the signal intensity reached a maximum value after 2 minutes (tₘ = 2 min.) and decreased thereafter, and the specific binding reaction reached equilibrium after 3 minutes; accordingly, time tₛ at which a saturation value was obtained, was 3 minute. Also, Line 26 showed that in the case of the urine having an hCG concentration of 2500 (IU/L), the signal intensity reached a maximum value after 1.5 minutes (tₘ = 1.5 min.) and decreased thereafter, and the specific binding reaction reached equilibrium after 5 minutes; accordingly, time tₛ at which a saturation value was obtained, was 5 minute. In other words, it was confirmed in these cases that there were regions where a maximum value was present and the change of the signal intensity was negative.

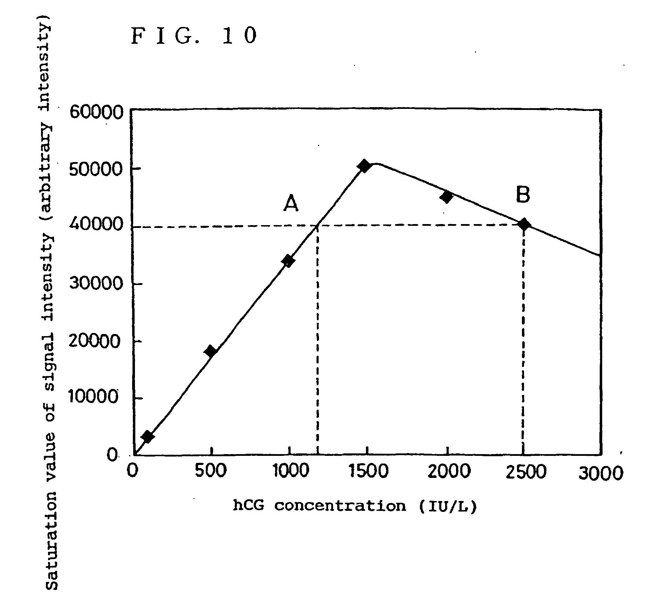

As such, some of the samples produced the above-described prozone phenomenon, and therefore, the relation between a saturation value of the signal intensity and the hCG concentration was determined next, and it was shown in FIG. 10. Since FIG. 9 showed that the signal intensity reached a saturation value 10 minutes after the urine was added dropwise to the sample application zone 3, the relation between the hCG concentration and the signal intensity measured 10 minutes after the urine was added dropwise, was shown in FIG. 10. FIG. 10 showed that the two concentrations, 1200 (IU/L) indicated by point A and 2500 (IU/L) indicated by point B, corresponded to a saturation value of 40000 of the signal intensity. This also showed that the urine having a concentration of 2500 (IU/L) produced the prozone phenomenon. That is, in this case, the hCG was excessively present in the urine and therefore the hCG not bound to the colloidal gold labeled a-hCG and the hCG bound to the colloidal gold labeled a-hCG competed to specifically bind to the a-hCG immobilized in the detection zone 5, thereby decreasing the amount of the colloidal gold labeled a-hCG bound in the detection zone 5 to decrease the signal intensity attributed to colloidal gold; accordingly, the signal intensity detected in the detection zone 5 did not reflect the amount of the hCG in the urine.

In the above-described manner, databases shown in FIGS. 9 and 10 were produced.

### Step (b)

Next, a urine 1 was collected from a test subject as a reagent to determine the hCG concentration in the urine of the test subject. Since the hCG as used herein was an analyte having the potential of exhibiting the prozone, a measurement pattern of the signal intensity corresponding to the urine was determined based on the database.

More specifically, since the urine 1 was considered to have the potential of exhibiting the prozone phenomenon, time tₛ at which a saturation value of the signal intensity was obtained was set to 8 minute, and time tₘ at which a maximum value of the signal intensity was obtained was set to 2 minute, and it was then determined to measure the signal intensity at time t₁ (= 2 min.), t₂ (= 3 min.) and tₛ (= 8 min.), wherein tₘ ≦ t₁ < t₂ ≦ tₛ was satisfied (first measurement pattern).

### Step (c)

The urine 1 collected in the step (b) was added dropwise to the sample application zone 3 of the strip 1 placed in the specific binding analysis apparatus shown in FIG. 7, thereby allowing the urine to permeate, by capillarity, into the retention zone 4 and then into the detection zone 5. Herein, it was anticipated that the hCG as the analyte in the urine 1 was specifically bound to the colloidal gold labeled a-hCG in the retention zone 4, followed by specifically binding to the immobilized a-hCG in the detection zone 5.

### Step (d)

Based on the first measurement pattern determined in the step (b), a signal intensity attributed to the specific binding reaction was measured at time t₁ (= 2 min.) and t₂ (= 3 min.) in a period in which the signal intensity had reached saturation; as a result, they were 49900 (A₁) and 40900 (A₂), respectively. Additionally, a saturation value of the signal intensity was measured at time tₛ, and it was 40000 (Aₛ).

### Step (e)

The signal intensity obtained in the step (d) gave (A₂- A₁)/(t₂ - t₁) = (49900 - 40900)/(2 - 3) = -9000, and therefore, the change of the signal intensity was negative. On the other hand, FIG. 10 indicated that the hCG concentrations corresponding to a saturation value of 40000 of the signal intensity were 1180 (IU/L) and 2500 (IU/L). Herein, since the change of the signal intensity was negative, it was able to determine the hCG concentration as 2500 (IU/L).

As described above, with the use of the specific binding analysis method and specific binding analysis apparatus in accordance with the present invention, it is possible to quantitatively or qualitatively determine an analyte in a sample in a rapid, simple and accurate manner with little influence of the prozone phenomenon attributed to the analyte as well as that of the background and foreign matter.

It should be noted that concentration was employed to determine the amount of an analyte in the foregoing, the unit representing the amount of an analyte is not limited to concentration. Any unit representing an amount can be employed in the present invention.

### Industrial Applicability

The specific binding analysis method in accordance with the present invention can be employed in turbidimetric immunoassay in which an sample containing an analyte is directly and specifically bound to a specific binding substance in a solution, as well as in analysis methods using chromatography.

Furthermore, with the use of the specific binding analysis method and specific binding analysis apparatus in accordance with the present invention, urinalysis and the like can be performed in an easy and simple manner, not only at hospitals but also at ordinary households.

## Claims

1. A specific binding analysis method of quantitatively determining an analyte in a sample from a signal attributed to a specific binding reaction between said analyte in said sample and a specific binding substance capable of specifically binding to said analyte, **characterized by** comprising the steps of:
(a) previously preparing a database comprising, for an individual sample containing a suspected analyte,
at least the relation between a concentration of said analyte and a saturation value or change of a signal intensity attributed to a specific binding reaction between said analyte in said sample and a specific binding substance capable of specifically binding to said analyte,
the change over time of said signal intensity, and
time tₛ at which said saturation value is obtained and time tₘ at which a maximum value of said signal intensity is obtained;
(b) preparing a sample containing an analyte and determining, based on said database, a measurement pattern of said signal intensity corresponding to said sample,
(c) causing a specific binding reaction between said analyte and a specific binding substance,
(d) measuring, based on said measurement pattern, said signal intensity at least twice in a period in which a signal intensity attributed to said specific binding reaction reaches saturation, after said step (c); and
(e) determining, based on said database, an amount of said analyte in said sample by using at least two signal intensities obtained in said step (d).

2. The specific binding analysis method in accordance with claim 1, **characterized in that**,
when said sample in said step (b) has said maximum value judging from said database,
said measurement pattern comprises measurement of signal intensities A₁ and A₂ at least at times t₁ and t₂ (tₘ ≦ t₁ < t₂ ≦ tₛ), respectively, and measurement of signal intensity Aₛ at said time tₛ, and that,
in said step (e), an amount of an analyte in said sample is determined, based on said relation in said database by using the change of said signal intensities A₁ and A₂, and said signal intensity Aₛ.

3. The specific binding analysis method in accordance with claim 1, **characterized in that**,
when said sample in said step (b) does not have said maximum value judging from said database,
said measurement pattern comprises measurement of signal intensities A₃ and A₄ at least at times t₃ and t₄ (t₃< t₄ < tₛ), and that,
in said step (e), an amount of an analyte in said sample is determined based on the change of said signal intensities A₃ and A4, and said relation in said database.

4. The specific binding analysis method in accordance with claim 1. **characterized in that**,
in said step (c), said analyte is specifically bound to a first specific binding substance labeled with a labeling material, and then said analyte is specifically bound to a second specific binding substance.

5. The specific binding analysis method in accordance with claim 4, **characterized by** further comprising,
prior to said step (b), step (X) of producing a strip comprising a sample application zone where said sample is applied and a detection zone where a second specific binding substance is practically immobilized and a signal attributed to a specific binding reaction can be detected,
wherein, in said step (d), said sample is applied to said sample application zone to allow said sample to flow into said detection zone by capillarity, thereby causing said analyte bound with said first specific binding substance to specifically bind to said second specific binding substance.

6. The specific binding analysis method in accordance with claim 4, **characterized in that**,
in said step (d), said signal intensity is measured by using said labeling material.

7. The specific binding analysis method in accordance with claim 5, **characterized in that,**
in said step (X), a retention zone containing a first specific binding substance labeled with a labeling material, is provided between said sample application zone and said detection zone.

8. The specific binding analysis method in accordance with claim 5, **characterized in that**,
in said step (d), said signal intensity is continuously measured in said detection zone, along the permeating direction of said sample on said strip.

9. The specific binding analysis method in accordance with claim 8, **characterized in that**,
in said step (d), said signal intensity is determined from an analytical line showing the relation between a position in said permeating direction and said signal intensity.

10. A specific binding analysis apparatus for quantitatively determining an analyte in a sample from a signal attributed to a specific binding reaction between said analyte and a specific binding substance capable of specifically binding to said analyte, **characterized by** comprising:
(1) a storage unit comprising a database which includes, for an individual sample containing a suspected analyte,
at least the relation between a concentration of said analyte and a saturation value or change of a signal intensity attributed to a specific binding reaction between said analyte and a specific binding substance capable of specifically binding to said analyte;
the change over time of said signal intensity; and
time tₛ at which said saturation value is obtained and time tₘ at which a maximum value of said signal intensity is obtained, each of which being measured from the start of said specific binding reaction;
(2) a strip comprising a sample application zone where a sample containing an analyte is applied and a detection zone where a specific binding substance is practically immobilized and a signal attributed to a specific binding reaction can be detected;
(3) a first detector which detects said signal;
(4) a control unit which determines, based on said database, a measurement pattern of said signal intensity and causes said detector to detect said signal according to said measurement pattern, thereby determining an intensity of said signal; and
(5) an analysis unit which determines, based on said database, an amount of said analyte in said sample by using said signal intensity.

11. The specific binding analysis apparatus in accordance with claim 10, **characterized by** further comprising a second detector which detects application of a sample to said sample application zone.

## Patentansprüche

1. Spezifisches Bindungsanalyseverfahren zum quantitativen Bestimmen eines zu bestimmenden Stoffes in einer Probe aus einem Signal, das einer spezifischen Bindungsreaktion zwischen dem zu bestimmenden Stoff in der Probe und einer spezifischen Bindungssubstanz, die an den zu bestimmenden Stoff spezifisch binden kann, zugeschrieben wird, **dadurch gekennzeichnet, dass** folgende Schritte umfasst sind:
(a) Vorheriges Herstellen einer Datenbank, die für eine individuelle Probe, die einen vermuteten zu bestimmenden Stoff enthält,
wenigstens die Beziehung zwischen einer Konzentration des zu bestimmenden Stoffes und eines Sättigungswertes oder Änderung einer Signalintensität, die einer spezifischen Bindungsreaktion zwischen dem zu bestimmenden Stoff in der Probe und einer spezifischen Bindungssubstanz, die spezifisch an den zu bestimmenden Stoff binden kann, zugeschrieben wird,
die Änderung über die Zeit der Signalintensität, und
die Zeit tₛ, bei welcher der Sättigungswert erhalten wird und die Zeit tₘ, bei welcher ein Maximalwert der Signalintensität erhalten wird;
(b) Herstellen einer Probe, die einen zu bestimmenden Stoff enthält und Bestimmen, basierend auf der Datenbank, eines Messmusters aus der Signalintensität, die der Probe entspricht,
(c) Verursachen einer spezifischen Bindungsreaktion zwischen dem zu bestimmenden Stoff und einer spezifischen Bindungssubstanz,
(d) Messen, basierend auf dem Messmuster, der Signalintensität wenigstens zweimal in einer Periode, in welcher eine Signalintensität, die der spezifischen Bindungsreaktion zugeschrieben wird, Sättigung erreicht, nach dem Schritt (c); und
(e) Bestimmen, basierend auf der Datenbank, einer Menge des zu bestimmenden Stoffes in der Probe, indem wenigstens zwei Signalintensitäten, die in dem Schritt (d) erhalten wurden, verwendet werden.

2. Spezifisches Bindungsanalyseverfahren gemäß Anspruch 1, **dadurch gekennzeichnet, dass**,
wenn die Probe in dem Schritt (b) den Maximalwertbesitz besitzt, der aus der Datenbank abgeschätzt wird,
das Messmuster einer Messung der Signalintensitäten A₁ und A₂ wenigstens zu jeweiligen Zeiten t₁ und t₂ (tₘ ≦ t₁ < t₂ ≦ tₛ) und Messung der Signalintensität Aₛ bei der Zeit tₛ umfasst, und, dass
in dem Schritt (e) eine Menge eines zu bestimmenden Stoffes in der Probe bestimmt wird, basierend auf den Zusammenhang zwischen der Datenbank, indem die Änderung der Signalintensitäten A₁ und A₂, und die Signalintensität Aₛ verwendet wird.

3. Spezifisches Bindungsanalyseverfahren gemäß Anspruch 1, **dadurch gekennzeichnet, dass**,
wenn die Probe in dem Schritt (b) nicht den Maximalwert besitzt, der aus der Datenbank abgeschätzt wird,
das Messmuster eine Messung der Signalintensitäten A₃ und A₄ wenigstens bei Zeiten t₃ und t₄ (t₃ < t₄ < tₛ) umfasst, und, dass
in dem Schritt (e) eine Menge eines zu bestimmenden Stoffes in der Probe basierend auf der Änderung der Signalintensitäten A₃ und A₄, und dem Zusammenhang in der Datenbank bestimmt wird.

4. Spezifisches Bindungsanalyseverfahren gemäß Anspruch 1, **dadurch gekennzeichnet, dass**
in dem Schritt (c) der zu bestimmende Stoff spezifisch an eine erste spezifische Bindungssubstanz gebunden wird, die mit einem Signaturmaterial versehen ist, und dann der zu bestimmende Stoff spezifisch an eine zweite spezifische Bindungssubstanz gebunden wird.

5. Spezifisches Bindungsanalyseverfahren gemäß Anspruch 4, **dadurch gekennzeichnet, dass** es ferner umfasst,
vor dem Schritt (b), Schritt (X) zum Herstellen eines Streifens, der eine Probenauftragungszone umfasst, wo die Probe aufgetragen wird und eine Detektionszone, wo eine zweite spezifische Bindungssubstanz praktisch immobilisiert wird und ein Signal, das einer spezifischen Bindungsreaktion zuschreibbar ist, detektiert werden kann,
wobei in dem Schritt (d) die Probe auf die Probenauftragungszone aufgetragen wird, um zu ermöglichen, dass die Probe in die Detektionszone durch Kapillarwirkung strömt, wodurch verursacht wird, dass der zu bestimmende Stoff, der an die erste spezifische Bindungssubstanz gebunden ist, spezifisch an die zweite spezifische Bindungssubstanz bindet.

6. Spezifisches Bindungsanalyseverfahren gemäß Anspruch 4, **dadurch gekennzeichnet, dass**
in dem Schritt (d) die Signalintensität gemessen wird, indem das Signaturmaterial verwendet wird.

7. Spezifisches Bindungsanalyseverfahren gemäß Anspruch 5, **dadurch gekennzeichnet, dass**
in dem Schritt (X), eine Retentionszone, die eine erste spezifische Bindungssubstanz enthält, die mit einem Signaturmaterial versehen ist, zwischen der Probenauftragungszone und der Detektionszone bereitgestellt wird.

8. Spezifisches Bindungsanalyseverfahren gemäß Anspruch 5, **dadurch gekennzeichnet, dass**
in dem Schritt (d) die Signalintensität kontinuierlich in der Detektionszone entlang der Eindringrichtung der Probe auf dem Streifen gemessen wird.

9. Spezifisches Bindungsanalyseverfahren gemäß Anspruch 8, **dadurch gekennzeichnet, dass**
in dem Schritt (d) die Signalintensität aus einer analytischen Linie bestimmt wird, die den Zusammenhang zwischen einer Position in der Eindringrichtung und der Signalintensität zeigt.

10. Spezifisches Bindungsanalysegerät zum quantitiven Bestimmen eines zu bestimmenden Stoffes in der Probe aus einem Signal, das einer spezifischen Bindungsreaktion zwischen dem zu bestimmenden Stoff und einer spezifischen Bindungssubstanz, die spezifisch an den zu bestimmenden Stoff binden kann, zurechenbar ist, **dadurch gekennzeichnet, dass** dieses umfasst:
(1) eine Speichereinheit, die eine Datenbank umfasst, welche für eine individuelle Probe, die einen vermuteten zu bestimmenden Stoff enthält, beinhaltet:
wenigstens den Zusammenhang zwischen einer Konzentration des zu bestimmenden Stoffes und eines Sättigungswertes oder einer Änderung einer Signalintensität, die einer spezifischen Bindungsreaktion zwischen dem zu bestimmenden Stoff und einer spezifischen Bindungssubstanz, die spezifisch an den zu bestimmenden Stoff binden kann, zuschreibbar ist;
die Änderung über die Zeit der Signalintensität;
Zeit tₛ, bei welcher der Sättigungswert erhalten wird und Zeit tₘ, bei welcher ein maximaler Wert der Signalintensität erhalten wird, welche jeweils vom Beginn der spezifischen Bindungsreaktion gemessen werden;
(2) ein Streifen, der eine Probe in Auftragungszone, wo eine Probe, die einen zu bestimmenden Stoff enthält, aufgetragen wird und eine Detektionszone, wo eine spezifische Bindungssubstanz praktisch immobilisiert ist und ein Signal, das einer spezifischen Bindungsreaktion zuschreibbar ist, detektiert werden kann, umfasst;
(3) einen ersten Detektor, welcher das Signal detektiert;
(4) eine Steuerungseinheit, welche basierend auf der Datenbank ein Messmuster der Signalintensität bestimmt und verursacht, dass der Detektor das dem Messmuster entsprechende Signal detektiert, wodurch eine Intensität des Signals bestimmt wird;
(5) eine Analyseeinheit, welche basierend auf der Datenbank eine Menge des zu bestimmenden Stoffes in der Probe bestimmt, indem die Signalintensität verwendet wird.

11. Spezifisches Bindungsanalysegerät gemäß Anspruch 10, **dadurch gekennzeichnet, dass** dieses ferner einen zweiten Detektor umfasst, welcher das Auftragen einer Probe auf die Probenauftragungszone detektiert.

## Revendications

1. Procédé d'analyse de liaison spécifique consistant à déterminer quantitativement un analyte dans un échantillon à partir d'un signal attribué à la réaction de liaison spécifique entre ledit analyte dans ledit échantillon et une substance de liaison spécifique capable de se lier spécifiquement au dit analyte, **caractérisé en ce qu'**il comprend les étapes consistant à :
(a) préparer précédemment une base de données comprenant, pour un échantillon individuel contenant un analyte suspect,
au moins la relation entre une concentration dudit analyte et une valeur de saturation ou de changement d'une intensité de signal attribuée à la réaction de liaison spécifique entre ledit analyte dans ledit échantillon et une substance de liaison spécifique capable de se lier spécifiquement au dit analyte,
le changement au cours du temps de ladite intensité du signal, et
le temps tₛ auquel ladite valeur de saturation est obtenue et le temps tₘ auquel la valeur maximale de ladite intensité du signal est obtenue ;
(b) préparer un échantillon contenant un analyte et déterminer, sur la base de ladite base de données, un motif de mesure pour ladite intensité de signal correspondant au dit échantillon,
(c) entraîner une réaction de liaison spécifique entre ledit analyte et une substance de liaison spécifique,
(d) mesurer, sur la base dudit motif de mesure, ladite intensité du signal au moins deux fois dans une période dans laquelle une intensité de signal attribuée à ladite réaction de liaison spécifique atteint la saturation, après ladite étape (c) ; et
(e) déterminer, sur la base de ladite base de données, une quantité dudit analyte dans ledit échantillon en utilisant au moins deux intensités de signal obtenues dans ladite étape (d).

2. Procédé d'analyse de liaison spécifique selon la revendication 1, **caractérisé en ce que**,
lorsque ledit échantillon dans ladite étape (b) présente ladite valeur maximale jugée depuis ladite base de données,
ledit motif de mesure comprend la mesure des intensités du signal A₁ et A₂ au moins aux temps t₁ et t₂ (tₘ ≤t₁ < t₂ ≤ tₛ) respectivement et la mesure de l'intensité de signal Aₛ au dit temps Tₛ, et **en ce que**,
dans ladite étape (e), une quantité d'analyte dans ledit échantillon est déterminée, sur la base de ladite relation dans ladite base de données en utilisant le changement desdites intensités de signal A₁ et A₂ et ladite intensité de signal Aₛ.

3. Procédé d'analyse de liaison spécifique selon la revendication 1, **caractérisé en ce que**,
lorsque ledit échantillon dans ladite étape (b) ne présente pas ledit jugement de valeur maximale depuis ladite base de données,
ledit motif de mesure comprend la mesure des intensités de signal A₃ et A₄ au moins au temps t₃ et t₄ (t₃ < t₄ < tₛ), et **en ce que**,
dans ladite étape (e), une quantité d'analyte dans ledit échantillon est déterminée sur la base du changement de ladite intensité de signal A₃ et A₄, et de ladite relation dans ladite base de données.

4. Procédé d'analyse de liaison spécifique selon la revendication 1, **caractérisé en ce que**,
dans ladite étape (c), ledit analyte est lié spécifiquement à une première substance de liaison spécifique marquée avec un matériau de marquage, et ensuite ledit analyte est lié spécifiquement à une seconde substance de liaison spécifique.

5. Procédé d'analyse de liaison spécifique selon la revendication 4, **caractérisé en ce qu'**il comprend en outre,
avant l'étape (b), l'étape (X) de production d'une bande comprenant une zone d'application d'échantillon où ledit échantillon est appliqué et une zone de détection où une seconde substance de liaison spécifique est immobilisée dans la pratique et un signal attribué à une réaction de liaison spécifique peut être détecté,
dans lequel, dans ladite étape (d), ledit échantillon est appliqué à la zone d'application d'échantillon pour permettre au dit échantillon de s'écouler dans ladite zone de détection par capillarité, entraînant de ce fait ladite liaison de l'analyte avec ladite première substance de liaison spécifique pour se lier spécifiquement à ladite seconde substance de liaison spécifique.

6. Procédé d'analyse de liaison spécifique selon la revendication 4, **caractérisé en ce que**,
dans ladite étape (d), ladite intensité de signal est mesurée en utilisant ledit matériau de marquage.

7. Procédé d'analyse de liaison spécifique selon la revendication 5, **caractérisé en ce que**,
dans ladite étape (X), une zone de rétention contenant une première substance de liaison spécifique marquée avec un matériau de marquage, est disposée entre ladite zone d'application d'échantillon et ladite zone de détection.

8. Procédé d'analyse de liaison spécifique selon la revendication 5, **caractérisé en ce que**,
dans ladite étape (d), ladite intensité de signal est mesurée continuellement dans ladite zone de détection, le long de la direction de perméation dudit échantillon sur ladite bande.

9. Procédé d'analyse de liaison spécifique selon la revendication 8, **caractérisé en ce que**,
dans l'étape (d), ladite intensité de signal est déterminée à partir d'une ligne analytique montrant la relation entre une position dans ladite direction de perméation et ladite intensité de signal.

10. Appareil d'analyse de liaison spécifique pour déterminer quantitativement un analyte dans un échantillon à partir d'un signal attribué à une réaction de liaison spécifique entre ledit analyte et une substance de liaison spécifique capable de se lier spécifiquement au dit analyte, **caractérisé en ce qu'**il comprend :
(1) une unité de mémorisation comprenant une base de données qui inclut, pour un échantillon individuel contenant un analyte suspect,
au moins la relation entre une concentration dudit analyte et une valeur de saturation ou de changement d'intensité de signal attribuée à une réaction de liaison spécifique entre ledit analyte et ladite substance de liaison spécifique capable de se lier spécifiquement au dit analyte ;
le changement au cours du temps de ladite intensité de signal ; et
le temps tₛ auquel ladite valeur de saturation est obtenue et le temps tₘ auquel une valeur maximale de ladite intensité de signal est obtenue, dont chacun est mesuré à partir du début de ladite réaction de liaison spécifique ;
(2) une bande comprenant une zone d'application d'échantillon où un échantillon contenant un analyte est appliqué et une zone de détection où une substance de liaison spécifique est immobilisée dans la pratique et un signal attribué à une réaction de liaison spécifique peut être détecté ;
(3) un premier détecteur qui détecte ledit signal ;
(4) une unité de commande qui détermine, sur la base de ladite base de données, un motif de mesure de ladite intensité de signal et amène ledit détecteur à détecter ledit signal en conformité avec ledit motif de mesure, déterminant de ce fait une intensité dudit signal ; et
(5) une unité d'analyse qui détermine, sur la base de ladite base de données, une quantité dudit analyte dans ledit échantillon en utilisant ladite intensité de signal.

11. Appareil d'analyse de liaison spécifique selon la revendication 9, **caractérisé en ce qu'**il comprend en outre un second détecteur qui détecte l'application d'un échantillon à ladite zone d'application d'échantillon.
